(19) Europäisches Patentamt
European Patent Office
Office européen des brevets

(11) Veröffentlichungsnummer: **0 334 014 A2**

# (12) EUROPÄISCHE PATENTANMELDUNG

(21) Anmeldenummer: 89102537.1

(22) Anmeldetag: 15.02.89

(51) Int. Cl.[4]: C07D 233/70 , C07D 233/84 , C07D 405/06 , A61K 31/415 , A61K 31/365

(30) Priorität: 25.02.88 DE 3805884
24.11.88 IT 2272288

(43) Veröffentlichungstag der Anmeldung: 27.09.89 Patentblatt 89/39

(84) Benannte Vertragsstaaten: AT BE CH DE ES FR GB GR IT LI LU NL SE

(71) Anmelder: BAYER AG
D-5090 Leverkusen 1 Bayerwerk(DE)

(72) Erfinder: Fey, Peter, Dr.
Ursulastrasse 14
D-5600 Wuppertal 1(DE)
Erfinder: Angerbauer, Rolf, Dr.
Sterntalerweg 29
D-5600 Wuppertal 1(DE)
Erfinder: Hüsch, Walter, Dr.
Am Eckbusch 43/50
D-5600 Wuppertal 1(DE)
Erfinder: Bischoff, Hilmar, Dr.
Am Rohm 78
D-5600 Wuppertal 1(DE)
Erfinder: Petzinna, Dieter, Dr.
Peter-Berten-Strasse 10
D-4000 Düsseldorf 21(DE)
Erfinder: Schmidt, Delf, Dr.
Am Eckbusch 55b
D-5600 Wuppertal 1(DE)
Erfinder: Thomas, Günter, Dr.
Via Campogallo 21/14
I-20020 Arese(IT)

## (54) Substituierte Imidazolinone und Imidazolinthione.

(57) Substituierte Imidazolinone und Imidazolinthione können durch Reduktion entsprechender Ketone und gegebenenfalls nach folgender Verseifung, Cyclisierung, Hydrierung und Isomerentrennung hergestellt werden. Die neuen Verbindungen können als Wirkstoffe in Arzneimittel verwendet werden.

EP 0 334 014 A2

## Substituierte Imidazolinone und Imidazolinthione

Die Erfindung betrifft substituierte Imidazolinone und Imidazolinthione, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP 22 478; US 4 231 938]. Darüberhinaus sind auch bestimmte Indolderivate bzw. Pyrazolderivate als Inhibitoren der HMG-CoA-Reduktase [EP-A 1 114 027; US-Patent 4 613 610] bekannt.

Es wurden substituierte Imidazolinone und Imidazolinthione der allgemeinen Formel (I)

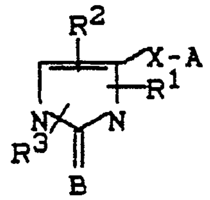

(I)

in welcher

$R^1$ - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$, $R^5$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin

$R^4$ und $R^5$ die oben angegebene Bedeutung haben, oder

$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

$R^3$ - für Wasserstoff oder

- für Acyl, Alkylsulfonyl oder Arylsulfonyl steht, wobei der Arylrest durch Alkyl oder Halogen substituiert sein kann, oder

- für Aminocarbonyl, Alkylaminocarbonyl, Dialkylaminocarbonyl, Arylaminocarbonyl oder Alkoxycarbonyl steht, oder

- für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$ und $R^5$ die oben angegebene Bedeutung haben, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

$R^3$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann.

worin

$R^4$ und $R^5$ die oben angegebene Bedeutung haben,

oder

$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

B - für Sauerstoff oder Schwefel steht,

X - für eine Gruppe der Formel $-CH_2-CH_2-$ oder $-CH=CH-$steht,

und

A - für eine Gruppe der Formel

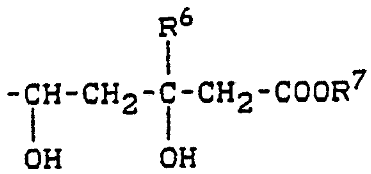 oder 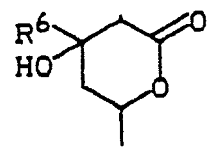 steht,

worin

$R^6$ - Wasserstoff oder Alkyl bedeutet und

$R^7$ - Wasserstoff,

- ein Alkyl-, Aralkyl- oder Aryl-Rest oder

- ein Kation bedeutet,

gefunden.

Überraschenderweise zeigen die erfindungsgemäßen substituierten Imidazolinone und Imidazolinthione eine bedeutende pharmakologische Wirkung, insbesondere eine gute inhibitorische Wirkung auf die HMG-CoA Reduktase (3-Hydroxy-3-methyl-glutaryl Coenzym A Reduktase).

Cycloalkyl steht im allgemeinen für einen cyclischen Kohlenwasserstoffrest mit 3 bis 8 Kohlenstoffatomen. Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Alkyl steht im allgemeinen für einen geradkettigen oder verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Me thylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niederalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Aryl steht im allgemeinen für einen aromatischen Rest mit 6 bis etwa 12 Kohlenstoffatomen. Bevorzugte Arylreste sind Phenyl, Naphthyl und Biphenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Al kylreste über eine $SO_2$-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethylsulfonyl und Phenylpropylsulfonyl.

Alkoxycarbonyl kann beispielsweise durch die Formel

$$-\underset{\underset{O}{\|}}{C}-OAlkyl$$

dargestellt werden. Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niederalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl im Rahmen der oben angebenen Definition steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Pyrazolyl, Indolyl, und Isoindolyl.

Falls $R^7$ für einen Alkyl, Aryl oder Aralkylrest steht, bildet sich eine Estergruppe.

Bevorzugt werden physiologisch verträgliche Ester, die der in vivo leicht zu einer freien Carboxylgruppe und einem entsprechenden physiologisch verträglichen Alkohol hydrolysiert werden. Hierzu gehören beispielsweise Alkylester ($C_1$ bis $C_6$) und Aralkylester ($C_7$ bis $C_{10}$), bevorzugt Niederalkylester sowie Benzylester. Insbesonders bevorzugt werden Methylester, Ethylester, Propylester und Benzylester.

Steht $R^7$ für ein Kation so, ist bevorzugt ein physiologisch verträgliches Metall-oder Ammoniumkation gemeint. Bevorzugt sind hierbei Alkali-bzw. Erdalkalikationen wie beispielsweise Natrium-, Kalium-, Magnesium- oder Calciumkationen, sowie Aluminium-oder Ammoniumkationen, sowie nicht-toxische substituierte Ammoniumkationen aus Aminen wie Diniederalkylamine, Triniederalkylamine, Dibenzylamin, N,N′-Dibenzylethylendiamin, N-Benzyl-β-phenylethylamin, N-Methylmorpholin oder N-Ethylmorpholin, Dihydroabiethylamin, N,N′-Bis-dihydroabiethylethylendiamin, N-Niederalkylpiperidin und andere Amine, die zur Bildung von Salzen verwendet werden können.

Im Rahmen der vorliegenden Erfindung entsprechen bevorzugt die substituierten Imidazolinone und Imidazolinthione der allgemeinen Formel

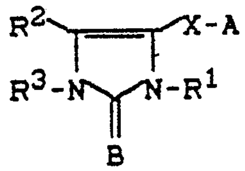

(Ia)

in welcher
$R^1$, $R^2$, $R^3$, X, A und B die oben angegebene Bedeutung haben,
und der allgemeinen Formel

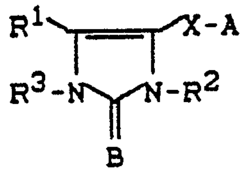

(Ib)

in welcher
$R^1$, $R^2$, $R^3$, X, A und B die oben angegebene Bedeutung haben.

Bevorzugt werden Imidazolinone und Imidazolinthione der Formel (I)

in welcher
$R^1$ - für Cyclopropyl, Cyclopentyl oder Cyclohexyl steht, oder für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$,
worin
$R^4$ und $R^5$ gleich oder verschieden sind und Niederalkyl, Phenyl, Benzyl, Acetyl, Benzoyl, Phenylsulfonyl oder Niederalkylsulfonyl bedeuten,
oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,
$R^2$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder
- für Phenyl oder Naphthyl steht, die bis zu 4-fach gleich oder verschieden substituiert sein können durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,
wobei
$R^4$ und $R^5$ die oben angegebene Bedeutung haben,
$R^3$ - für Wasserstoff oder
- für Benzoyl oder Niederalkylcarbonyl steht, oder
- für Niederalkylsulfonyl,Phenylsulfonyl oder Tolylsulfonyl steht, oder
- für Aminocarbonyl, Niederalkylaminocarbonyl, Diniederalkylaminocarbonyl, Phenylaminocarbonyl oder Niederalkoxycarbonyl steht, oder
- für Cyclopropyl, Cyclopentyl oder Cyclohexyl steht oder
- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$,

worin
$R^4$ und $R^5$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei dei genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können, oder
$R^3$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl steht, oder
$R^3$ - für Phenyl oder Naphthyl steht, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenylethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,
wobei
$R^4$ und $R^5$ die oben angegebene Bedeutung haben
B - für eine Gruppe der Formel O oder S steht,
X - für eine Gruppe der Formel $-CH_2-CH_2-$ oder $-CH=CH-$steht,
A - für eine Gruppe der Formel

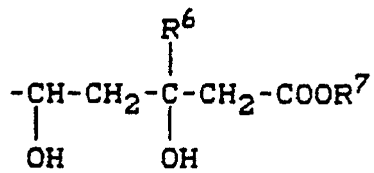 oder 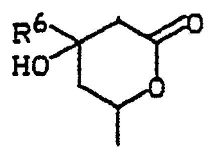 steht,

worin
$R^5$ - Wasserstoff oder Niederalkyl bedeutet, und
$R^7$ - Alkyl ($C_1$ bis $C_6$), Aryl ($C_6$ bis $C_{12}$) oder Aralkyl ($C_7$ bis $C_{10}$) bedeutet, oder
- ein physiologisch verträgliches Kation bedeutet.

Besonders bevorzugt sind Verbindungen der allgemeinen Formeln (I)
in welcher
$R^1$ - für Cyclopropyl, Cyclopentyl oder Cyclohexyl steht, oder
- für Methyl, Ethyl, Propyl, Isopropyl, Butyl, sec.Butyl oder tert.Butyl steht, die substituiert sein können durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
$R^2$ - für Pyridyl, Pyrimidyl, Chinolyl oder Isochinolyl steht, das durch Fluor, Chlor, Methyl, Methoxy oder Trifluormethyl substituiert sein kann, oder
- für Phenyl steht, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.-Butoxycarbonyl,
$R^3$ - für Wasserstoff, Cyclopropyl, Cyclopentyl oder Cyclohexyl steht, oder
- für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Brom, Cyano, Methoxy, Ethoxy, Propoxy, Isopropoxy, Butoxy, Isobutoxy, tert.-Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl,

oder durch eine Gruppe $-NR^4R^5$,
wobei
$R^4$ und $R^5$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolin, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
$R^3$ - für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
$R^3$ - für Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^4R^5$ substituiert sein kann,
wobei
$R^4$ und $R^5$ die oben angegebene Bedeutung haben,
oder
$R^3$ - für Benzoyl, Acetyl, Ethylcarbonyl, Propylcarbonyl, Isopropylcarbonyl, Methylsulfonyl, Ethylsulfonyl, Propylsulfonyl, Isopropylsulfonyl, Butylsulfonyl, Isobutylsulfonyl, tert.Butylsulfonyl, Phenylsulfonyl oder Tolylsulfonyl steht,
- für Aminocarbonyl, Methyl-, Ethyl-, Propyl-, Isopropyl-, Butyl-, Isobutyl- oder tert.Butylaminocarbonyl, Dimethyl-, Diethyl-, Dipropyl-, Diisopro pyl-, Dibutyl- oder Diisobutylaminocarbonyl, Phenylaminocarbonyl,Methoxycarbonyl, Ethoxycarbonyl, Propoxycarbonyl, Isopropoxycarbonyl, Butoxycarbonyl, Isobutoxycarbonyl oder tert.Butoxycarbonyl steht,
B - für Sauerstoff oder Schwefel steht,
X - für eine Gruppe der Formel $-CH_2-CH_2-$ oder $-CH=CH-$steht,
A - für eine Gruppe der Formel

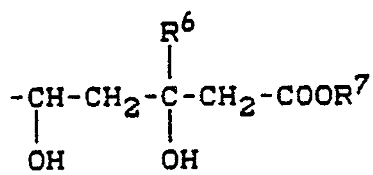 oder 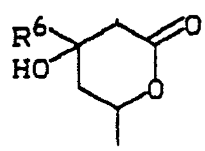 steht,

worin
$R^6$ - Wasserstoff, Methyl, Ethyl, Propyl, Isopropyl, Butyl, Isobutyl oder tert.Butyl bedeutet, und
$R^7$ - Wasserstoff, Methyl, Ethyl, Propyl, Isopropyl, Butyl, Isobutyl, tert.Butyl, oder Benzyl bedeutet, oder ein Natrium-, Kalium-, Calcium- oder Magnesium- oder Ammoniumkation bedeutet.

Die erfindungsgemäßen substituierten Imidazolinone und Imidazolinthione der allgemeinen Formel (I) haben mehrere asymmetrische Kohlenstoffatome und können daher in verschiedenen stereochemischen Formen existieren, Sowohl die einzelnen Isomeren als auch deren Mischungen sind Gegenstand der Erfindung.

Je nach der Bedeutung der Gruppe X bzw. des Restes A ergeben sich unterschiedliche Stereoisomere, die im folgenden näher erläutert werden sollten:

a) Steht die Gruppe -X- für eine Gruppe der Formel $-CH=CH-$ , so können die erfindungsgemäßen Verbindungen in zwei stereoisomeren Formen existieren, die an der Doppelbindung E-konfiguriert (II) oder Z-konfiguriert (III) sein können:

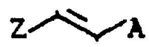 (II)

(E-konfiguriert)

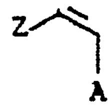 (III)

(Z-konfiguriert)

wobei
Z für den Rest

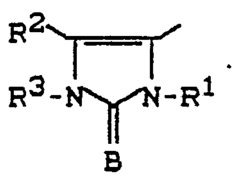 oder 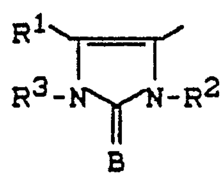 steht.

Bevorzugt sind diejenigen Verbindungen der allgemeinen Formel (I) die E-konfiguriert sind (II).

b) Steht der Rest -A- für eine Gruppe der Formel

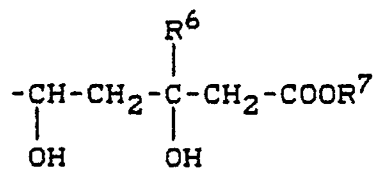

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

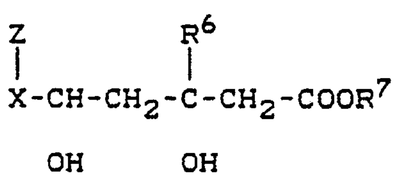 Erythro-Form (IV)

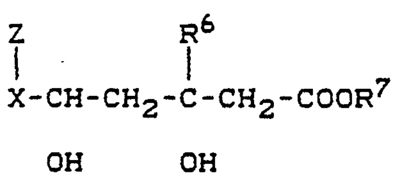 Threo-Form (V).

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S.5S-Isomeres (Threo-Form).

Bevorzugt sind hierbei die Erythro-konfigurierten Isomeren, besonders bevorzugt das 3R,5S-Isomere sowie das 3R,5S-3S,5R-Racemat.

c) Steht der Rest -A- für eine Gruppe der Formel

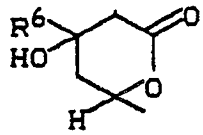

so besitzen die substituierten Imidazolinone und Imidazolinthione mindestens zwei asymmetrische Kohlenstoffatome, nämlich das Kohlenstoffatom an dem die Hydroxygruppe gebunden ist, und das Kohlenstoffatom an welchem der Rest der Formel Z-X- gebunden ist.

Je nach der Stellung der Hydroxygruppe zur freien Valenz am Lactonring können die substituierten Imidazolinone und Imidazolinthione als cis-Lactone (VI) oder als trans-Lactone (VII) vorliegen:

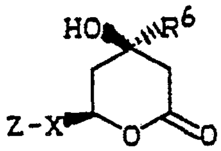

cis-Lacton (VI)

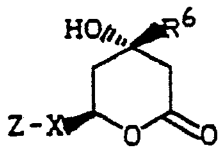

trans-Lacton (VII).

Sowohl vom cis-Lacton aus als auch vom trans-Lacton existieren wiederum jeweils zwei Isomeren nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Imidazolinone und Imidazolinthione genannt:

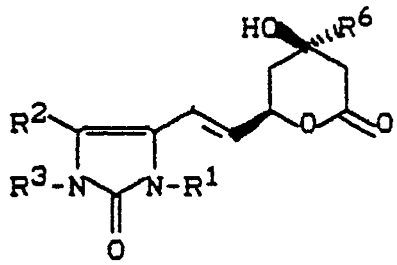

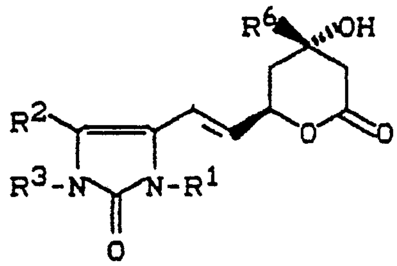

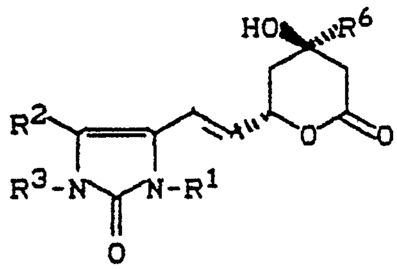

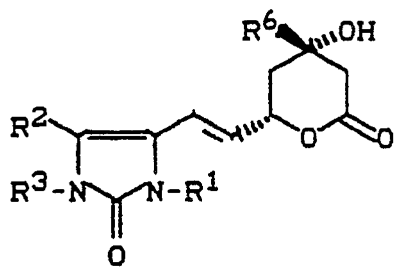

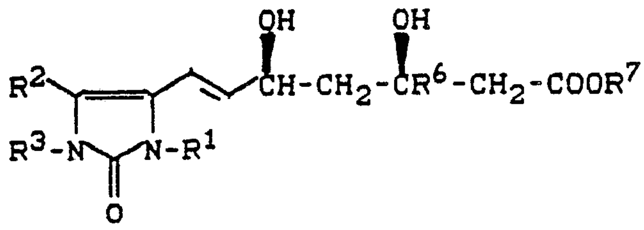

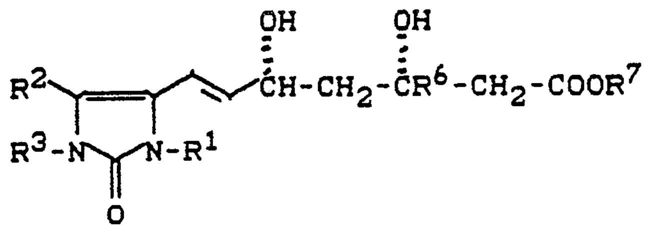

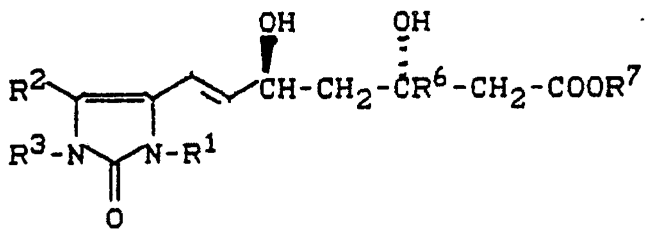

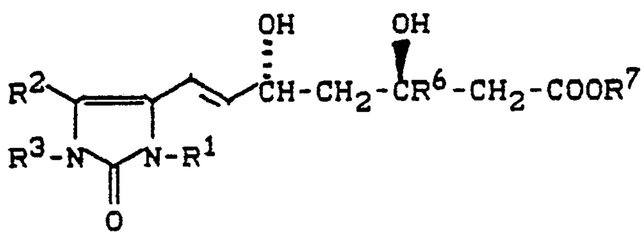

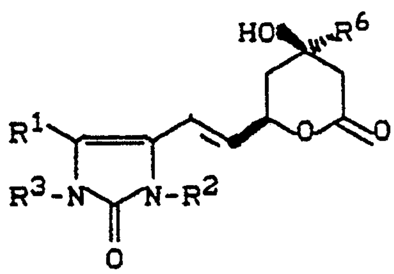

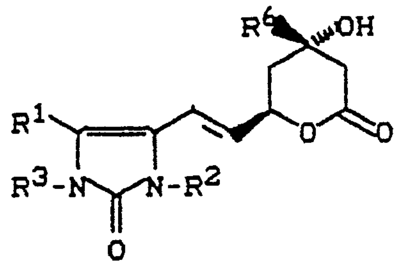

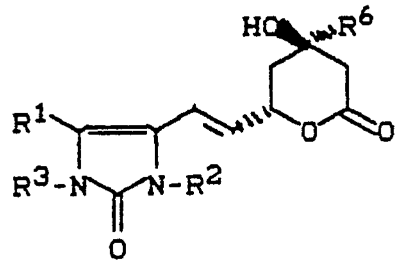

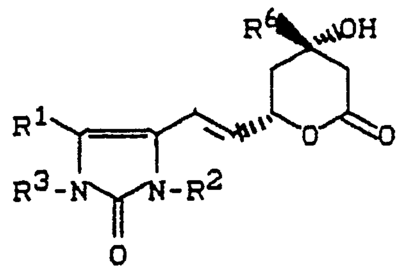

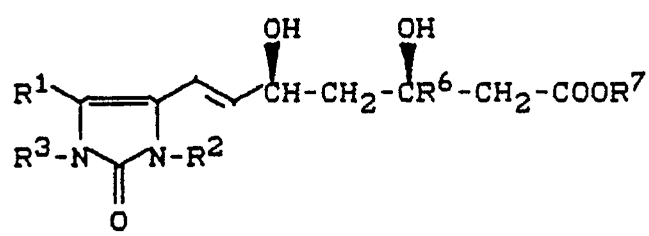

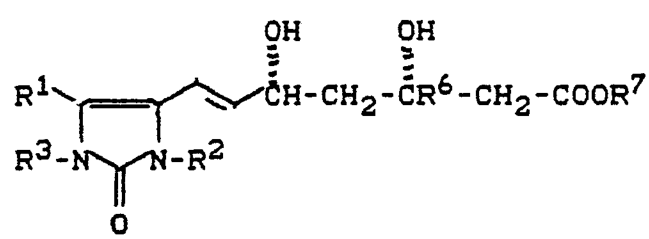

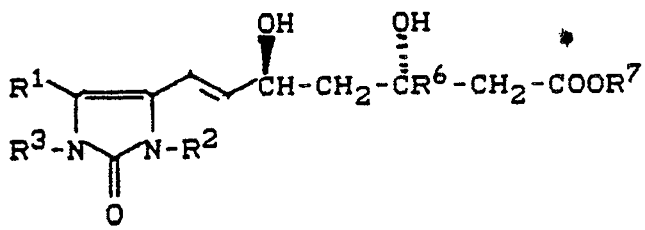

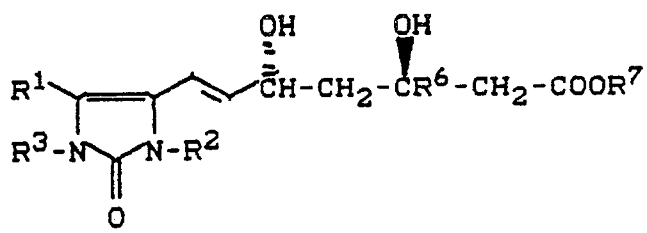

Ganz besonders bevorzugt sind die Verbindungen der allgemeinen Formel (Ia) und (Ib) in welchen

$R^1$ - für Cyclopropyl, Methyl, Ethyl, Propyl, Isopropyl, Butyl, Isobutyl oder tert.-Butyl steht.

$R^2$ - für Phenyl steht, das bis zu 2-fach gleich oder verschieden durch Methyl oder Trifluormethyl substituiert sein kann,

$R^3$ - für Methyl, Isopropyl, tert.-Butyl steht oder

- für Phenyl steht, das bis zu 2-fach gleich oder verschieden durch Methyl, Methoxy, Fluor oder Chlor substituiert sein kann,

B - für Sauerstoff oder Schwefel steht,

X - für eine Gruppe der Formel

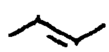 (E-konfiguriert) steht

und

A - für eine Gruppe der Formel

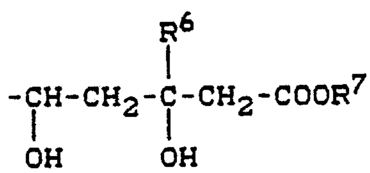 oder 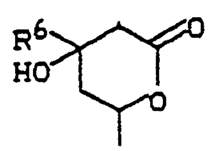 steht,

worin

$R^6$ - Wasserstoff bedeutet und

$R^7$ - Wasserstoff, Methyl oder Ethyl bedeutet oder ein Natrium- oder Kaliumkation bedeutet.

Außerdem wurde ein Verfahren zur Herstellung der substituierten Imidazolinone und Imidazolinthione der allgemeinen Formel (I)

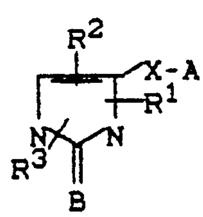 (I)

in welcher
$R^1$, $R^2$, $R^3$, A, B und X die oben genannte Bedeutung haben,
gefunden,
das dadurch gekennzeichnet ist, daß man
Ketone der allgemeinen Formel (VIII)

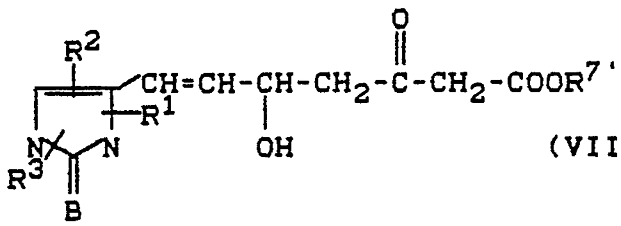

(VIII)

in welcher
$R^1$, $R^2$, $R^3$ und B die oben angegebene Bedeutung haben, und
$R^{7'}$ - für Alkyl ($C_1$ bis $C_6$), Aryl ($C_6$ bis $C_{12}$) oder Aralkyl ($C_7$ bis $C_{10}$), steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = $-CH=CH-$) nach üblichen Methoden hydriert,
und gegebenenfalls Isomeren trennt.

Das erfindungsgemäße Verfahren kann durch das folgendes Formelschema erläutert werden:

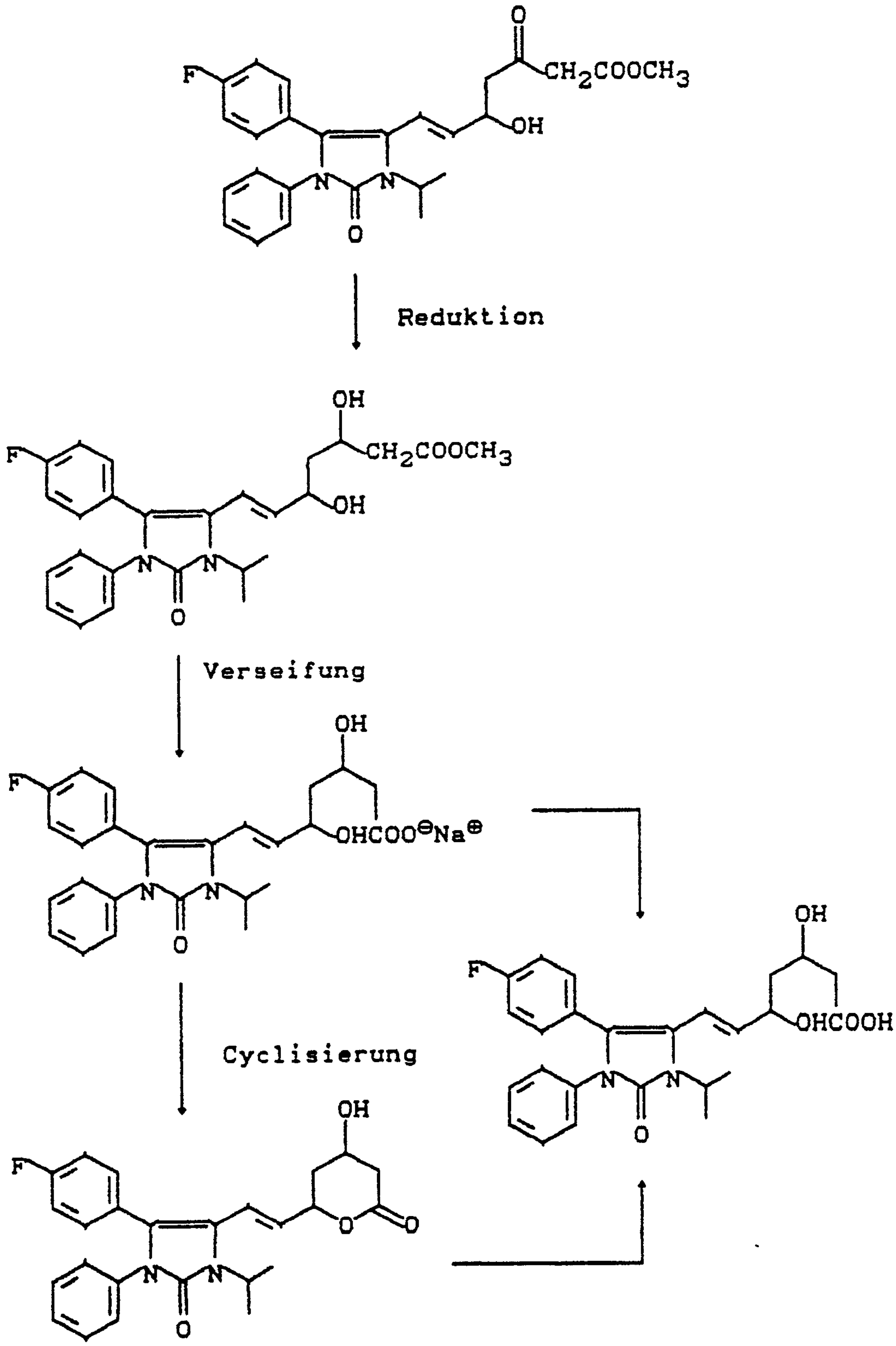


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Zylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels weise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80° C bis Raumtemperatur (30° C), bevorzugt von -78° C bis 0° C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Darüber hinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

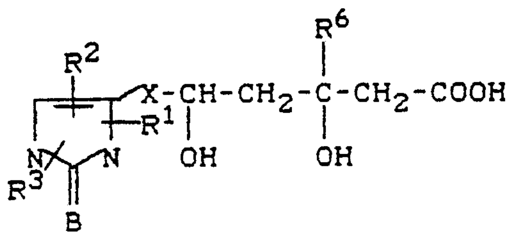
(Ic)

in welcher

$R^1$, $R^2$, $R^3$, $R^6$, B und X die oben angegebene Bedeutung haben.

Die Carbonsäureester im Rahmen der allgemeinen Formel (I) entsprechen der Formel (Id)

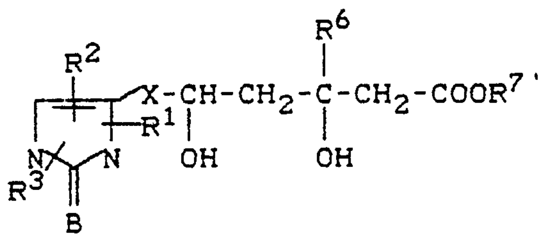
(Id)

in welcher

$R^1$, $R^2$, $R^3$, $R^6$, B und X die oben angegebene Bedeutung haben, und

$R^{7'}$ für Alkyl, Aralkyl oder Aryl steht.

Die Salze der erfindungsgemäßen Verbindungen im Rahmen der allgemeinen Formel (I) entsprechen der Formel (Ie)

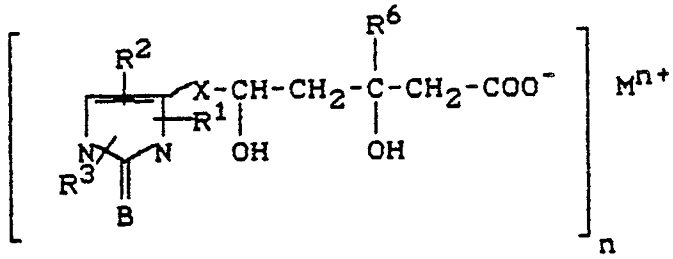

(Ie)

in welcher
$R^1$, $R^2$, $R^3$, $R^6$, B und X die oben angegebene Bedeutung haben,
und
$M^n$ für ein n-wertiges Kation steht, worin n 1 oder 2 bedeutet.

Die Lactone im Rahmen der allgemeinen Formel (I) entsprechen der Formel (If)

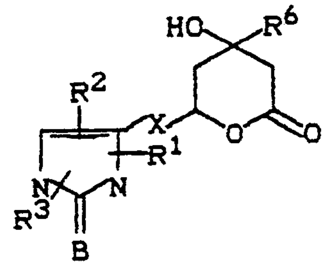

(If)

in welcher
$R^1$, $R^2$, $R^3$, $R^6$, B und X die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Druchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischensprodukte, die isoliert werden können. Die erfindungsgemäßen Säuren (Ic) erhält man durch Behandeln der Salze (Ie) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäueren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40° C bis +200° C, bevorzugt von -25° C bis +50° C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüber hinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Dicyclohexylcarbodiimid-Paratoluolsulfonat, N-Cyclohexyl-N′-[2-(N″-methylmorpholinium)ethyl]carbodiimid oder N-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0° C bis +80° C, bevorzugt von +10° C bis +50° C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Lactone. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig)

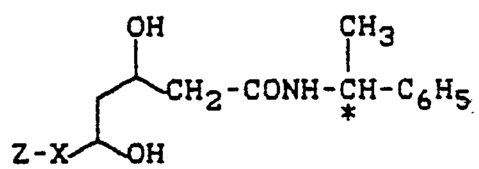

(Ig)

wobei

Z und X die oben angegebene Bedeutung haben,

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kalium hydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukt nach der oben beschriebenen Methode abhängig ist von der Konfiguration der Ausgangsstoffe.

Die Isomerentrennung soll im folgenden Schema beispielhaft erläutert werden:

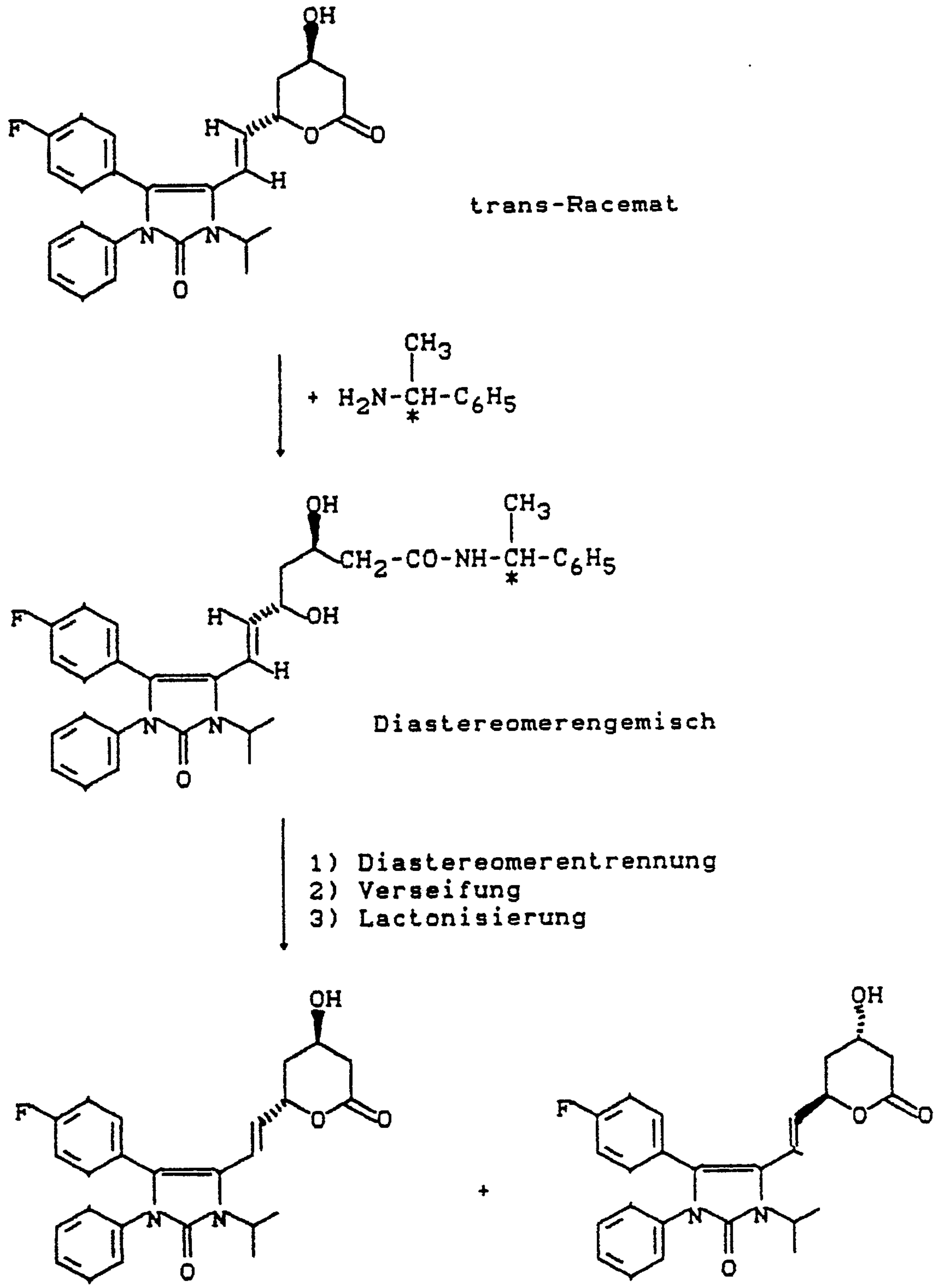


Die als Ausgangsstoffe eingesetzten Ketone (VIII) sind neu.
Es wurde ein Verfahren zur Herstellung der erfindungsgemäßen Ketone der allgemeinen Formel (VIII)

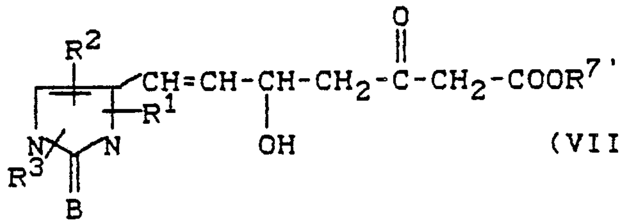

(VIII)

in welcher
$R^{1'}$, $R^2$, $R^3$ und $R^{7'}$ die oben genannte Bedeutung haben, gefunden, das dadurch gekennzeichnet ist, daß man Aldehyde der allgemeinen Formel (IX)

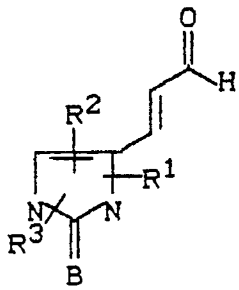

(IX)

in welcher
$R^{1'}$, $R^2$, $R^3$ und B die oben angegebene Bedeutung haben,
in inerten Lösemitteln mit Acetessigester der allgemeinen Formel (X)

$$H_3C-\overset{\overset{O}{||}}{C}-CH_2-COOR^{7'} \qquad (X)$$

in welcher
$R^{7'}$ die oben angegebene Bedeutung hat,
in Anwesenheit von Basen umsetzt.

Das erfindungsgemäße Verfahren kann beispielsweise durch folgendes Formelschema erläutert werden:

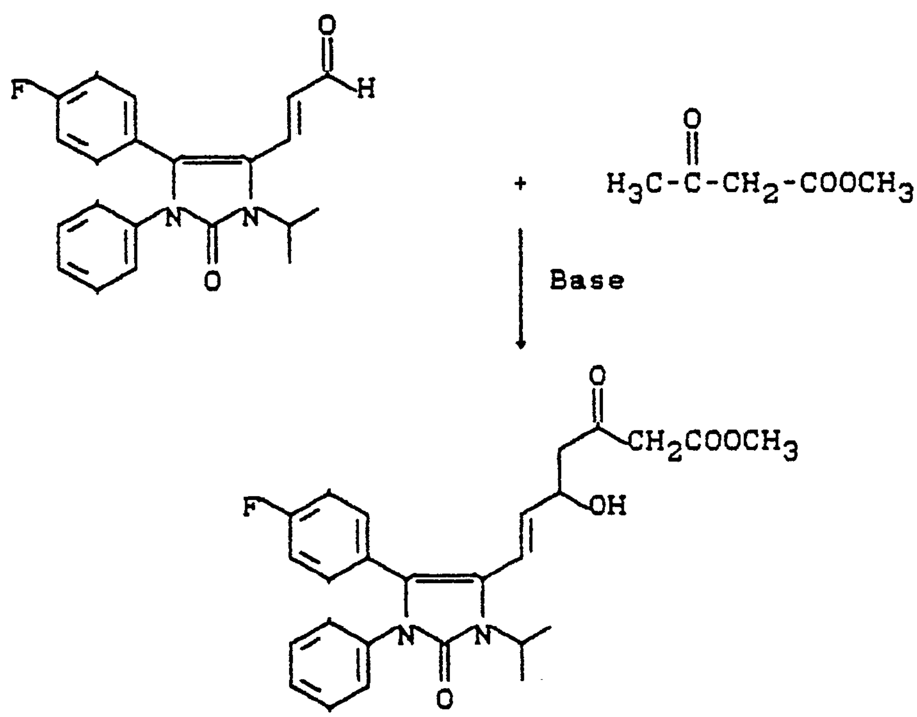

Als Basen kommen hierbei die üblichen stark basischen Verbindungen in Frage. Hierzu gehören bevorzugt lithium-organische Verbindungen wie beispielsweise N-Butyllithium, sec.Butyllithium, tert.Butyllithium oder Phenyllithium, oder Amide wie beispielsweise Lithiumdiisopropyl amid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Eventuell sind Zusätze von Metallhalogeniden wie z.B. Magnesiumchlorid oder Zinkbromid vorteilhaft. Besonders bevorzugt ist der Zusatz von Zinkbromid.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die als Ausgangsstoffe eingesetzten Aldehyde der allgemeinen Formel (IX) sind neu.

Es wurde außerdem ein Verfahren zur Herstellung der Aldehyde der allgemeinen Formel (IX)

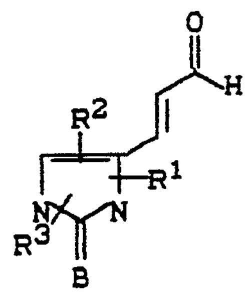 (IX)

in welcher
$R^1$, $R^2$, $R^3$ und B die oben genannte Bedeutung haben,
gefunden, das dadurch gekennzeichnet ist, daß man Imidazolinone und Imidazolinthione der allgemeinen Formel (XI)

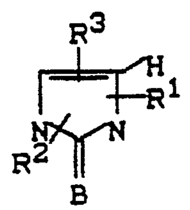 (XI)

in welcher
$R^1$, $R^2$, $R^3$ und B oben angegebene Bedeutung haben,
in inerten Lösemitteln in Anwesenheit von Hilfsstoffen mit N,N-Dialkylaminoacrolein der Formel (XII)

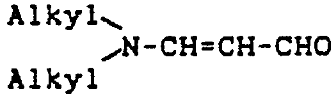

(XII),

wobei
Alkyl für einen geradkettigen oder verzweigten Kohlenwasserstoffrest mit 1 bis 6 Kohlenstoffatomen steht, umsetzt.

Das erfindungsgemäße Verfahren kann beispielsweise durch folgendes Formelschema verdeutlicht werden:

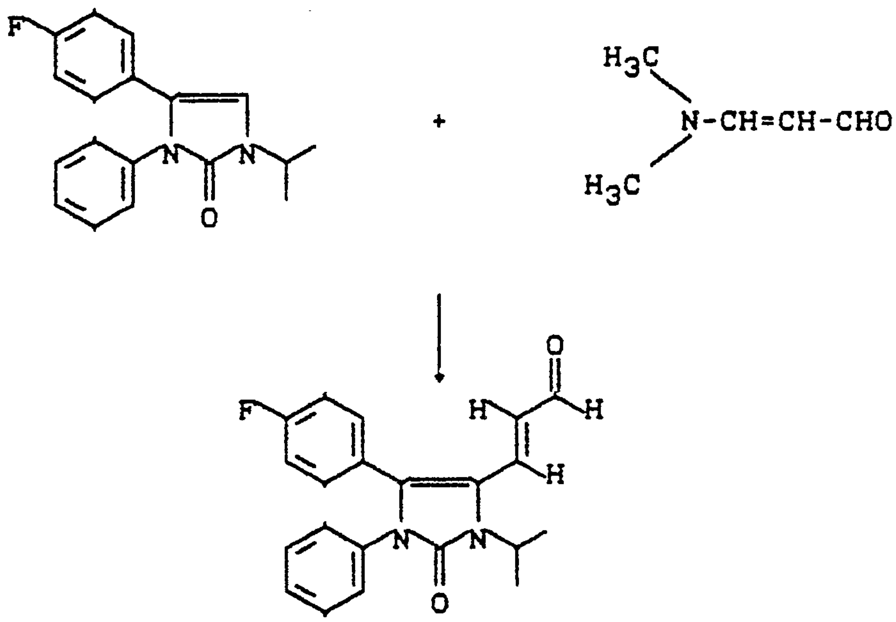

Als Lösemittel eignen sich hierbei die üblichen organischen Lösemittel, die unter den Reaktionsbedingungen stabil sind. Hierzu gehören bevorzugt Kohlenwasserstoffe wie Benzol, Toluol, Xylol, Hexan, Erdölfraktionen, Chlorbenzol oder Dichlorbenzol, oder Ether wie Diethylether, Dioxan oder Tetrahydrofuran, oder Chlorkohlenwasserstoffe wie Methylenchlorid, Chloroform oder Tetrachlorkohlenstoff oder Acetonitril. Ebenso ist es möglich, Gemische der genannten Lösemittel einzusetzen. Besonders bevorzugt wird wasserfreies Acetonitril oder Chloroform verwendet.

Als Hilfsstoffe werden im allgemeinen Säurechloride verwendet. Bevorzugt wird Phosphoroxychlorid oder Phosgen, besonders bevorzugt Phosphoroxychlorid eingesetzt.

Die Reaktion wird in einem Temperaturbereich von -20°C bis +150°C, bevorzugt von 0°C bis +100°C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung des Verfahrens wird das Dimethylaminoacrolein im allgemeinen in einer Menge von 2 bis 6, bevorzugt von 3 bis 4 mol, bezogen auf 1 mol des Pyrrols eingesetzt.

Die als Ausgangsstoffe eingesetzten Imidazolinone und Imidazolinthione der allgemeinen Formel (XI) sind bekannt oder können nach bekannten Methoden ausgehend von Aminoketonen hergestellt werden [EP-A-222 644].

Die als Ausgangsstoffe eingesetzten Aminoketone sind bekannt oder können nach bekannten Methoden hergestellt werden [Methoden der organischen Chemie, Vo. 7/2c, 2251 ff (1977); J. Org. Chem. 33, 494 (1968)].

Die erfindungsgemäßen Verbindungen der allgemeinen Formel (I) besitzen wertvolle pharmakologische Eigenschaften und können in Arzneimittel eingesetzt werden. Insbesondere sind sie Inhibitoren der 3-Hydroxy-3-methyl-glutarylCoenzym A (HMG-CoA) Reduktase und infolge dessen Inhibitoren der Cholesterolbiosynthese. Sie können deshalb zur Behandlung von Hyperlipoproteinämie, Lipoproteinämie oder Arteriosklerose eingesetzt werden.

Die neuen Wirkstoffe können in bekannter Weise in übliche Formulierungen überführt werden, wie

Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Beispiel 1

2-(Benzyl-methylamino)-1-(4-fluorphenyl)-1-oxo-ethanhydrochlorid

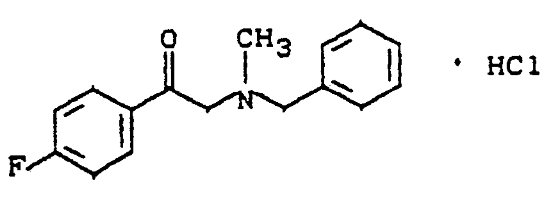

Zu einer Lösung von 81,8 g (0,68 mol) N-Methylbenzylamin in 220 ml Diethylether tropft man bei 0° C eine Lösung von α-Brom-4-fluoracetphenon (70,9 g, 0,33 mol) in 310 ml Diethylether und rührt über Nacht. Der Niederschlag wird abgetrennt, das Filtrat mit Salzsäuregas versetzt, restliches Methylbenzylaminhydrochlorid abgetrennt, das Filtrat mit Aceton verrührt, wobei das Produkt ausfällt.
Ausbeute: 52,8 g 55 % der Theorie
$^1$H-NMR (DMSO): δ - 2,85 (s, 3H, $CH_3$); 4,5 (br, 2H, $CH_2$); 5,1 (br, 2H, $CH_2C=O$); 7,3-8,2 (m, 9H, Aromaten-H) ppm.

Beispiel 2

1-(4-Fluorphenyl)-2-methylamino-1-oxo-ethanhydrochlorid

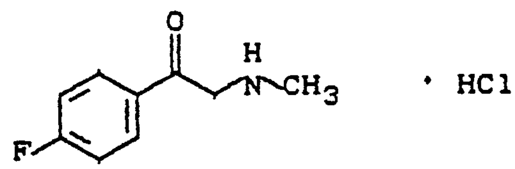

Zu einer Lösung von 10 g (31 mmol) der Verbindung aus Beispiel 1 in 200 ml Eisessig gibt man unter Stickstoffatmosphäre 1,2 g 10 % Palladium auf Kohle. Nach der Hydrierung in einer Schüttelapparatur (Wasserstoffaufnahme ca. 700 ml) wird der Katalysator abfiltriert, das Lösungsmittel abdestilliert, das Produkt mit Wasser gewaschen und getrocknet.
Ausbeute: 7,2 g 97 % der Theorie
'H-NMR (DMSO): δ = 2,6 (s, 3H, $CH_3$); 4,75 (s, 2H, $CH_2$); 7,3-8,2 (m, 4H, Aromaten H); 9,5 (br, 2H, NH) ppm.

Beispiel 3

N-[2-(4-Fluorphenyl)-2-oxo-ethyl]-N-methyl-N′-phenylharnstoff

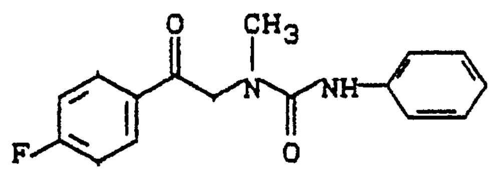

Zu einer Suspension von 3 g (12,5 mmol) der Verbindung aus Beispiel 2 in 30 ml Toluol gibt man 1,26 g (12,5 mmol) Phenylisocyanat und tropft bei 0° C langsam 1,5 g (12,5 mmol) Triethylamin hinzu. Es wird über Nacht bei 25° C gerührt. Die Toluolphase wird mit Wasser gewaschen, wobei das Produkt ausfällt. Nach dem Trocknen erhält man 3,54 g.
Ausbeute: 98,9 % der Theorie
'H-NMR (DMSO): δ = 2,8 (s, 2H, $CH_2$); 3,35 (s, 3H, $CH_3$); 3,6 (s, 1H, NH); 6,9-7,6 (m, 9H, Aromaten-H) ppm.

Beispiel 4

4-(4-Fluorphenyl)-1-methyl-3-phenyl-4-imidazolin-2-on

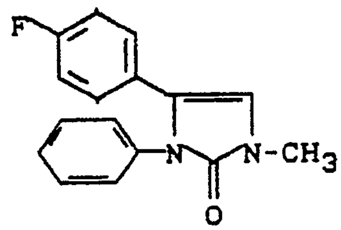

In 50 ml Eisessig werden 3,5 g (12,2 mmol) der Verbindung aus Beispiel 3 24 Stunden unter Rückfluß erhitzt, das Lösungsmittel wird entfernt und der Rückstand mit Wasser gewaschen. Nach dem Trocknen erhält man 3,12 g.
Ausbeute: 95,3 % der Theorie
$^1$H-NMR (DMSO): $\delta$ = 3,3 (s, 3H, $CH_3$); 6,9 (s, 1H, Imidazol-H); 7,0-7,5 (m, 9H, Aromaten-H) ppm.

Beispiel 5

(E)-3-[4-(4-Fluorphenyl)-1-methyl-3-phenyl-4-imidazolin-2-on-5-yl]-prop-2-enal

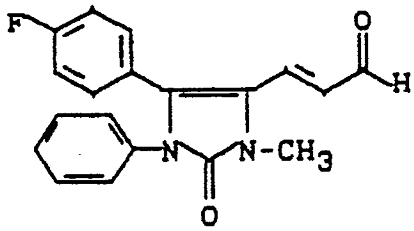

Zu 1,37 ml (15 mmol) Phosphoroxychlorid in 8,5 ml Acetonitril tropft man bei 0°-5° C innerhalb 10 min unter Stickstoffatmosphäre 1,54 ml (14 mmol) N,N-Dimethylaminoacrolein in 5,3 ml Acetonitril. Nach 10 min gibt man eine Lösung von 1,3 g (5 mmol) der Verbindung aus Beispiel 4 in 10 ml Acetonitril hinzu und erhitzt über Nacht zum Sieden. Bei 10° C gibt man 3,7 g Natriumhydroxid in 50 ml Wasser/50 ml Toluol hinzu, wäscht die wäßrige Phase nochmals mit Toluol, trocknet die organischen Phasen und erhält nach Kristallisation aus Ether 0,85 g.
Ausbeute: 51,2 % der Theorie
$^1$H-NMR ($CDCl_3$): $\delta$ = 3,6 (s, 3H, $CH_3$); 6,5 (dd, 1H, CH-CHO); 7,0-7,5 (m, 10H, AromatenH, CH); 9,5 (d, 1H, CHO) ppm.

Beispiel 6

Methyl-(E)-7-[4-(4-fluorphenyl)-1-methyl-3-phenyl-4-imidazolin-2-on-5-yl]-5-hydroxy-3-oxo-hept-6-enoat

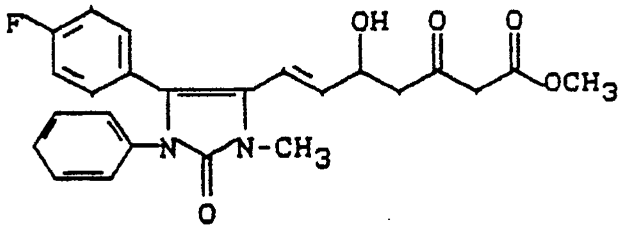

Unter Stickstoff tropft man zu einer Suspension von 135 mg (5,6 mmol) Natriumhydrid in 14 ml Tetrahydrofuran bei -5 °C 0,59 ml (5,4 mmol) Acetessigsäuremethylester.

Nach 15 min tropft man bei -5 °C 4,0 ml (5,6 mmol) 15 %iges n-Butyllithium in n-Hexan hinzu, rührt 15 min addiert eine Lösung von 1,3 g (5,9 mmol) Zinkbromid in 20 ml THF, rührt 15 min und addiert eine Lösung von 0,6 g (1,8 mmol) der Verbindung aus Beispiel 5 in 6 ml Tetrahydrofuran. Nach 30 min bei -5 °C hydrolysiert man mit gesättigter Ammoniumchloridlösung, wäscht dreimal mit Dichlormethan, trocknet die organische Phase und erhält nach Entfernen des Lösungsmittels und Waschen des Rückstandes mit Petrolether 380 mg.

Ausbeute: 48 % der Theorie

$^1$H-NMR ($CDCl_3$): δ = 2,7; 3,0 (2d, 2H, $CH_2$); 3,5 (s, 5H, $NCH_3$, $CH_2C=O$); 3,7 (s, 3H, $OCH_3$); 4,65 (br, 1H, CHOH); 5,9 (dd, 1H, <u>CH</u>CHO); 6,4 (d, 1H, CH); 6,9-7,4 (m, 9H, Aromaten-H) ppm.

## Beispiel 7

Methyl-erythro-(E)-3,5-dihydroxy-7-[4-(4-fluorphenyl)-1-methyl-3-phenyl-4-imidazolin-2-on-5-yl]-hept-6-enoat

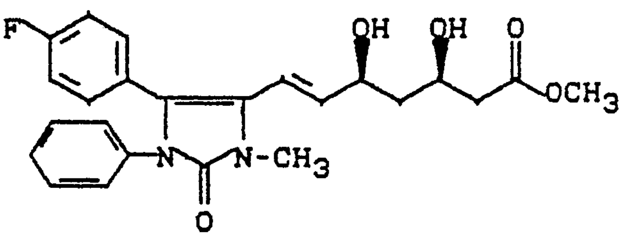

Durch eine Lösung von 350 mg (0,8 mmol) der Verbindung aus Beispiel 6 und 1,12 ml (1,12 mmol) Triethylboran (1 m in Tetrahydrofuran) in 7 ml Tetrahydrofuran bläst man 5 min Luft, gibt bei -30 °C 42,3 mg (1,12 mmol) Natriumborhydrid und langsam 0,76 ml Methanol hinzu, rührt 30 min bei -30 °C, gibt ein Gemisch von 3 ml 30 %iger Wasserstoffperoxidlösung und 6,5 ml Wasser hinzu, daß die Temperatur 0 °C nicht übersteigt, verdünnt nach 30 min mit Wasser, wäscht dreimal mit Essigester, wäscht die organische Phase mit Natriumhydrogencarbonatlösung, trocknet, entfernt das Lösungsmittel im Vakuum und erhält nach Chromatographie über Kieselgel (Essigester) 60 mg.

Ausbeute: 17 % der Theorie

$^1$H-NMR ($CDCl_3$): δ = 1,6 (m, 2H, $CH_2$); 2,5 (m, 2H, $CH_2C=O$); 3,4 (s, 3H, $NCH_3$); 3,7 (s, 3H, $CH_3$); 4,2 (br, 1H, CHOH); 4,4 (br, 1H, CHOH); 5,9 (dd, 1H, <u>CH</u>CHOH); 6,3 (d, 1H, CH); 6,8-7,5 (m, 9H, Aromaten-H) ppm.

## Beispiel 8

2-(Benzyl-isopropylamino)-1-(4-fluorphenyl)-1-oxo-1-ethan-hydrochlorid

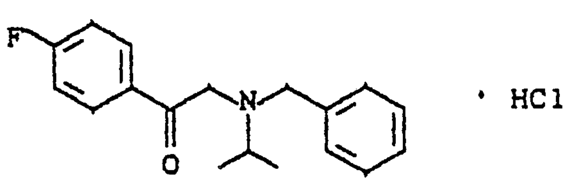

Analog Beispiel 1 erhält man aus 55 g (0,25 mol) α-Brom-4-fluoracetphenon und 78 g (0,52 mol) Isopropylbenzylamin, 46,9 g 2-(Benzyl-isopropylamino)- -1-(4-fluor- phenyl))-1-oxo-1-ethan-hydrochlorid.

Ausbeute: 57,5 % der Theorie

$^1$H-NMR (DMSO): δ = 1,4 (dd, 6H, $CH_3$); 3,8 (m, 1H, CH); 4,4 (m, 2H, $CH_2$); 5,0 (m, 2H, $CH_2C=O$); 7,2-8,0 (m, 9H, Aromaten-H) ppm.

Beispiel 9

1-(4-Fluorphenyl)-2-isopropylamino-1-oxo-ethan-hydrochlorid

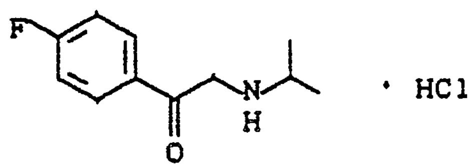

Analog Beispiel 2 erhält man aus 44,8 g (0,14 mol) der Verbindung aus Beispiel 8 25,7 g 1-(4-Fluorphenyl)-2-isopropylamino-1-oxo-ethan-hydrochlorid.
Ausbeute: 79,8 % der Theorie
$^1$H-NMR (DMSO): $\delta$ = 1,3 (d, 6H, $CH_3$); 3,4 (m, 1H, CH); 4,8 (br, 2H, $CH_2$); 7,4-8,3 (m, 4H, Aromaten-H) ppm.

Beispiel 10

N-[2-(4-Fluorphenyl)-2-oxo-ethyl]-N-isopropyl-N′-phenylharnstoff

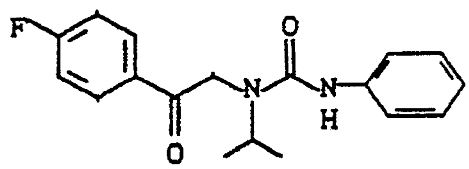

Analog Beispiel 3 erhält man aus 7,0 g (30,2 mmol) der Verbindung aus Beispiel 9 und 3,05 g (30,2 mmol) Phenylisocyanat 6,3 g N-[2-(4-Fluorphenyl)-2-oxo-ethyl]-N-isopropyl-N′-phenyl-harnstoff.
Ausbeute: 66,4 % der Theorie
$^1$H-NMR ($CDCl_3$): $\delta$ = 1,2 (dd, 6H, $CH_3$); 2,9 (s, 1H, NH); 3,5 (dd, 2H, $CH_2$); 4,35 (m, 1H, CH); 6,9-7,6 (m, 9H, Aromaten-H) ppm.

Beispiel 11

4-(4-Fluorphenyl)-1-isopropyl-3-phenyl-4-imidazolin-2-on

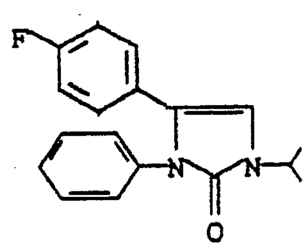

Analog Beispiel 4 erhält man aus 5,3 g (16,9 mmol) der Verbindung aus Beispiel 10 4,6 g 4-(4-Fluorphenyl)-1-isopropyl-3-phenyl-4-imidazolin-2-on.
Ausbeute: 91,9 % der Theorie

$^1$H-NMR ($CDCl_3$): δ = 1,4 (d, 6H, $CH_3$); 4,5 (m, 1H, CH); 6,45 (s, 1H, CH); 6,8-7,4 (m, 9H, Aromaten-H) ppm.

Beispiel 12

(E)-3-[4-(4-Fluorphenyl)-1-isopropyl-3-phenyl-4-imidazolin-2-on-5-yl]-prop-2-enal

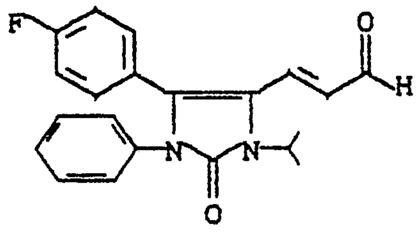

Analog Beispiel 5 erhält man aus 2,0 g (6,8 mmol) der Verbindung aus Beispiel 11 1,6 g (E)-3-[4-(4-Fluorphenyl)-1-isopropyl-3-phenyl-4-imidazolin-2-on-5-yl]-prop-2-enal.
Ausbeute: 67,7 % der Theorie
$^1$H-NMR ($CDCl_3$): δ = 1,6 (d, 6H, $CH_3$); 4,6 (m, 1H, CH); 6,2 (dd, 1H, CHCHO); 6,9-7,4 (m, 10H, Aromaten-H, CH); 9,4 (d, 1H, CHO) ppm.

Beispiel 13

Methyl-(E)-7-[4-(4-fluorphenyl)-1-isopropyl-3-phenyl-4-imidazolin-2-on-5-yl]-5-hydroxy-3-oxo-hept-6-enoat

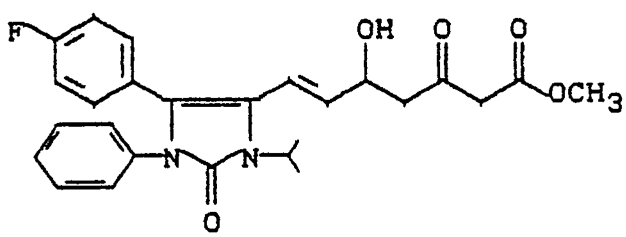

Analog Beispiel 6 erhält man aus 0,8 g (2,3 mmol) der Verbindung aus Beispiel 12 1,1 g Rohprodukt, nach Chromatographie über Kieselgel (Dichlormethan/Methanol 10:1) erhält man 0,8 g Methyl-(E)-7-[4-(4-fluorphenyl)-1-isopropyl-3-phenyl-4-imidazolin-2-on-5-yl]-5-hydroxy-3-oxo-hept-6-enoat.
Ausbeute: 75 % der Theorie
$^1$H-NMR ($CDCl_3$): δ = 1,6 (d, 6H, $CH_3$); 2,7; 3,0 (2d, 2H, $CH_2$); 3,4 (s, 2H, $CH_2$); 3,75 (s, 3H, $CH_3$); 4,4 (m, 1H, CH); 4,6 (m, 1H, CH-OH); 5,6 (dd, 1H, CH); 6,4 (d, 1H, CH); 6,8-7,4 (m, 9H, Aromaten-H) ppm.

Beispiel 14

Methyl-erythro-(E)-3,5-dihydroxy-7-[4-(4-fluorphenyl)-1-isopropyl-3-phenyl-4-imidazolin-2-on-5-yl]-hept-6-enoat

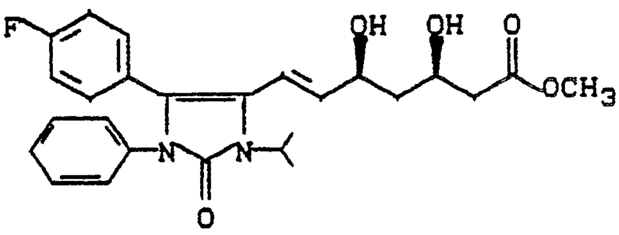

Analog Beispiel 7 erhält man aus 0,8 g (1,7 mol) der Verbindung aus Beispiel 13 320 mg Methyl-erythro-(E)-3,5-dihydroxy-7-[4-(4-fluorphenyl)-1-isopropyl-3-phenyl-4-imidazolin-2-on-5-yl]-hept-6-enoat.
Ausbeute: 40 % der Theorie
$^1$H-NMR ($CDCl_3$): $\delta$ = 1,5 (m, 8H, $CH_3$, $CH_2$); 2,5 (m, 2H, $CH_2$); 3,7 (s, 3H, $CH_3$); 4,2; 4,4 (2m, 2H, CH); 4,5 (m, 1H, CH); 5,6 (dd, 1H, CH); 6,4 (d, 1H, CH); 6,8-7,3 (m, 9H, Aromaten-H) ppm.

Beispiel 15

N-[2-(4-Fluorphenyl)-2-oxo-ethyl]-N-methyl-N′-phenylthioharnstoff

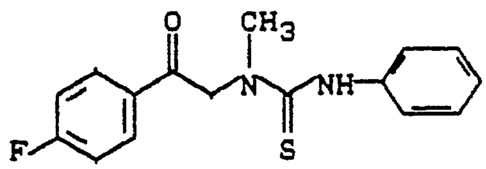

Analog Beispiel 3 erhält man aus 3 g (12,5 mmol) der Verbindung aus Beispiel 2 und 1,6 g (12,5 mmol) Phenylisothiocyanat 3,67 g der obengenannten Verbindung.
Ausbeute: 97,2 % der Theorie
$^1$H-NMR (DMSO): $\delta$ = 2,3 (s, 1H); 3,35 (s, 3H); 4,0 (q, 1H); 7,0-7,6 (m, 5H) ppm.

Beispiel 16

4-(4-Fluorphenyl)-1-methyl-3-phenyl-4-imidazolin-2-thion

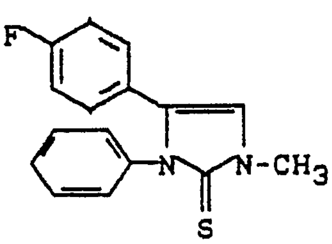

Analog Beispiel 4 erhält man aus 3,5 g (11,6 mmol) der Verbindung aus Beispiel 15 2,99 g der obengenannten Verbindung.
Ausbeute: 91 % der Theorie
$^1$H-NMR ($CDCl_3$): $\delta$ = 3,7 (s, 3H); 6,8-7,5 (m, 10H) ppm.

Beispiel 17

(E)-3-[4-(4-Fluorphenyl)-1-methyl-3-phenyl-4-imidazolin-2-thion-5-yl]-prop-2-enal

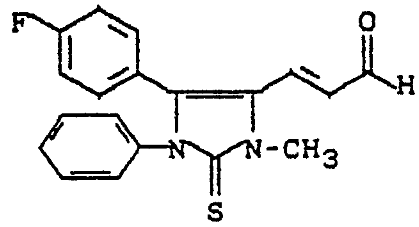

Analog Beispiel 5 erhält man aus 2,4 g (8,45 mmol) der Verbindung aus Beispiel 16 1,8 g Produkt.
Ausbeute: 61,1 % der Theorie
$^1$H-NMR ($CDCl_3$): δ = 4,0 (s, 3H); 6,5 (dd, 1H); 6,9 (d, 1H); 6,95-7,5 (m, 9H); 9,5 (d, 1H) ppm.

Beispiel 18

Methyl-(E)-7-[4-(4-fluorphenyl)-1-methyl-3-phenyl-4-imidazolin-2-thion-5-yl]-5-hydroxy-3-oxo-hept-6-enoat

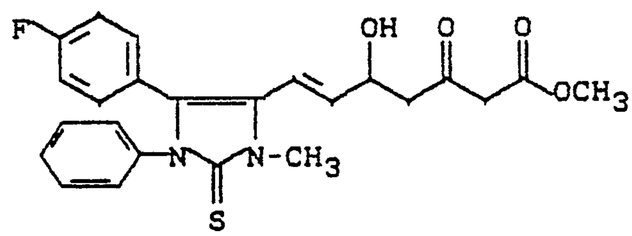

Analog Beispiel 6 erhält man aus 1,8 g (5,3 mmol) der Verbindung aus Beispiel 17 3,0 g Rohprodukt.
Ausbeute: 100 % der Theorie
$^1$H-NMR ($CDCl_3$): δ = 2,3 (s, 2H); 3,5 (s, 3H); 3,7 (s, 3H); 3,75 (s, 2H): 4,65 (m, 1H); 5,85 (dd, 1H); 5,9 (d, 1H); 6,8-7,5 (m, 9H);

Beispiel 19

Methyl-erythro-(E)-3,5-dihydroxy-7-[4-(4-fluorphenyl)-1-methyl-3-phenyl-4-imidazolin-2-thion-5-yl]-hept-6-enoat

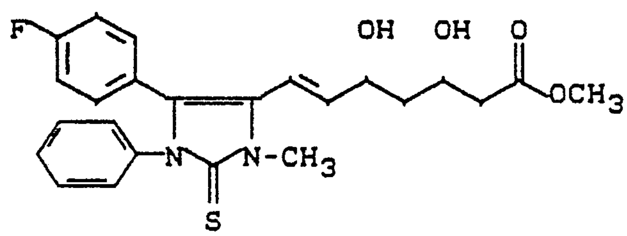

Analog Beispiel 7 erhält man aus 2,11 g (4,6 mmol) der Verbindung aus Beispiel 18 0,3 g Produkt.
Ausbeute: 14,2 % der Theorie
$^1$H-NMR ($CDCl_3$): δ 1,6 (m, 2H); 2,5 (d, 2H); 3,7 (s, 3H); 3,8 (s, 3H); 4,2 (m, 1H); 4,5 (m, 1H); 5,85 (dd, 1H);

5,9 (d, 1H); 6,9-7,4 (m, 9H) ppm.

Beispiel 20

Methyl-erythro-(E)-3,5-dihydroxy-7-[4-(4-fluorphenyl)-1-isopropyl-3-phenyl-4-imidazolin-2-thion-5-yl]-hept-6-enoat

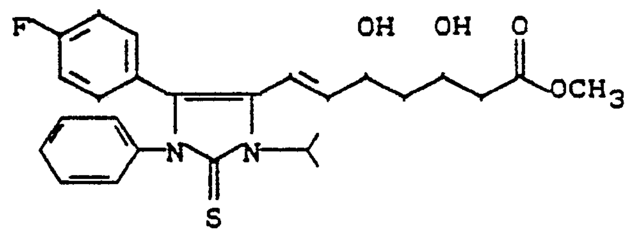

Die Verbindung aus Beispiel 20 kann in Analogie zu Beispiel 19 erhalten werden.

Anwendungsbeispiele

Beispiel 1

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15 000* g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes mit dem SPE-Puffer aufgenommen, homogenisiert und bei -78° C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37° C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase, 35 µMol $K_xH_y$-Phosphat pH 7,2, 20 µl Enzympräparation und 56 nMol 3-Hydroxy-3-methyl-glutaryl Coenzym A (Glutaryl-3-$^{14}$C) 100 000 dpm.

Nach einer Inkubation von 60 Minuten bei 37° C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml eines Scintillationslösungsmittels versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 100 gesetzt und mit dem simultan bestimmten $IC_{50}$-Wert der Testverbindung verglichen.

Die Wirkstoffe nach den Beispielen 1 bis 14 zeigen im Vergleich zu Mevinolin eine höhere Wirkung.

Beispiel 2

Die Serum-Cholesterin-senkende Wirkung der erfindungsgemäßen Verbindungen auf die Blutcholesterinwerte von Hunden und Meerschweinchen wurde in mehrwöchigen Fütterungsexperimenten gefunden. Dazu wurde die zu untersuchende Substanz im Fall der Hundeversuche über einen mehrwöchigen Zeitraum einmal täglichh in einer Kapsel gesunden Beagle Hunden zusammen mit dem Futter p.o. gegeben. Dem Futter war außerdem während der gesamten Versuchsperiode, d.h. vor, während und nach der Applikatonsperiode der zu untersuchenden Substanz Colestyramin (4 g/100 g Futter) als Gallensäuresequestrant beigemischt.

Zweimal wöchentlich wurde den Hunden venöses Blut abgenommen und das Serumcholesterin mit einem handelsüblichen Testkit enzymatisch bestimmt. Die Serumcholesterinwerte während der Applikationsperiode wurden mit den Serumcholesterinwerten vor der Applikationsperiode (Kontrollen) verglichen.

Den Meerschweinchen wurde die Substanz in das Futter gemischt. Nach zwei Wochen wurde den Tieren Blut entnommen und das Serum-Cholesterin bestimmt.

## Ansprüche

1. Substituierte Imidazolinone und Imidazolinthione der allgemeinen Formel

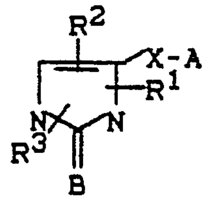

in welcher

$R^1$ - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$, $R^5$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin

$R^4$ und $R^5$ die oben angegebene Bedeutung haben,

oder

$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

$R^3$ - für Wasserstoff oder

- für Acyl, Alkylsulfonyl oder Arylsulfonyl steht, wobei der Arylrest durch Alkyl oder Halogen substituiert sein kann, oder

- für Aminocarbonyl, Alkylaminocarbonyl, Dialkylaminocarbonyl, Arylaminocarbonyl oder Alkoxycarbonyl steht, oder

- für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin
$R^4$ und $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
$R^3$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,
worin
$R^4$ und $R^5$ die oben angegebene Bedeutung haben,
oder
$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutungdhaben,
B - für Sauerstoff oder Schwefel steht,
X - für eine Gruppe der Formel $-CH_2-CH_2-$ oder $-CH=CH-$steht,
und
A - für eine Gruppe der Formel

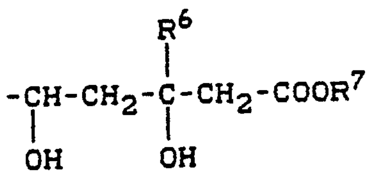 oder 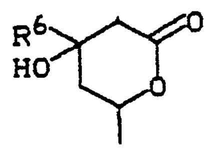 steht,

worin
$R^6$ - Wasserstoff oder Alkyl bedeutet
und
$R^7$ - Wasserstoff,
- einen Methyl-, Aralkyl- oder Aryl-Rest oder
- ein Kation bedeutet.

2. Substituierte Imidazolinone und Imidazolinthione nach Anspruch 1
worin
$R^1$ - für Cyclopropyl, Cyclopentyl oder Cyclohexyl steht, oder
- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$,
worin
$R^4$ und $R^5$ gleich oder verschieden sind und Niederalkyl, Phenyl, Benzyl, Acetyl, Benzoyl, Phenylsulfonyl oder Niederalkylsulfonyl bedeuten,
oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,
$R^2$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder
- für Phenyl oder Naphthyl steht, die bis zu 4-fach gleich oder verschieden substituiert sein können durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,
wobei
$R^4$ und $R^5$ die oben angegebene Bedeutung haben,
$R^3$ - für Wasserstoff oder
- für Benzoyl oder Niederalkylcarbonyl steht, oder
- für Niederalkylsulfonyl,Phenylsulfonyl oder Tolylsulfonyl steht, oder
- für Aminocarbonyl, Niederalkylaminocarbonyl, Diniederalkylaminocarbonyl, Phenylaminocarbonyl oder Niederalkoxycarbonyl steht, oder
- für Cyclopropyl, Cyclopentyl oder Cyclohexyl steht oder
- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$,
worin
$R^4$ und $R^5$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können, oder
$R^3$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, die bis zu 2-fach gleioh oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder
$R^3$ - für Phenyl oder Naphthyl steht, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,
wobei
$R^4$ und $R^5$ die oben angegebene Bedeutung haben
B - für eine Gruppe der Formel O oder S steht,
X - für eine Gruppe der Formel $-CH_2-CH_2-$ oder $-CH=CH-$steht, und
A - für eine Gruppe der Formel

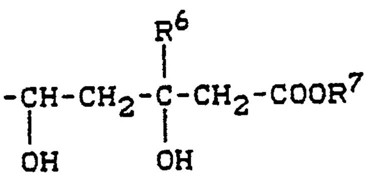

oder

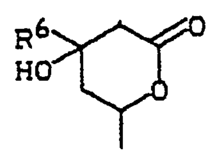

steht,

worin
$R^6$ - Wasserstoff oder Niederalkyl bedeutet, und
$R^7$ - Alkyl ($C_1$ bis $C_6$), Aryl ($C_6$ bis $C_{12}$) oder Aralkyl ($C_7$ bis $C_{10}$) bedeutet, oder
- ein physiologisch verträgliches Kation bedeutet.

3. Substituierte Imidazolinone und Imidazolinthione nach den Ansprüchen 1 und 2,
worin
$R^1$ - für Cyclopropyl, Cyclopentyl oder Cyclohexyl steht, oder
- für Methyl, Ethyl, Propyl, Isopropyl, Butyl, sec.-Butyl oder tert.Butyl steht, die substituiert sein können durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
$R^2$ - für Pyridyl, Pyrimidyl, Chinolyl oder Isochinolyl steht, die durch Fluor, Chlor, Methyl, Methoxy oder

Trifluormethyl substituiert sein können, oder 0 für Phenyl stehen, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,

$R^3$ - für Wasserstoff, Cyclopropyl, Cyclopentyl oder Cyclohexyl steht, oder

- für Methyl, Ethyl, Propyl, Isopropyl, Butyl, Isobutyl, tert.Butyl, Pentyl, Isopentyl, Hexyl oder Isohexyl steht, die substituiert sein können durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^4R^5$,

wobei

$R^4$ und $R^5$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

oder durch Pyridyl, Pyrimidyl, Pyrazinyl, Pyridazinyl, Chinolin, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder

$R^3$ - für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder

$R^3$ - für Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^4R^5$ substituiert sein kann,

wobei

$R^4$ und $R^5$ die oben angegebene Bedeutung haben,

oder

$R^3$ - für Benzoyl, Acetyl, Ethylcarbonyl, Propylcarbonyl, Isopropylcarbonyl, Methylsulfonyl, Ethylsulfonyl, Propylsulfonyl, Isopropylsulfonyl, Butylsulfonyl, Isobutylsulfonyl, tert.Butylsulfonyl, Phenylsulfonyl oder Tolylsulfonyl steht,

- für Aminocarbonyl, Methyl-, Ethyl-, Propyl-, Isopropyl-, Butyl-, Isobutyl- oder tert.Butylaminocarbonyl, Dimethyl-, Diethyl-, Dipropyl-, Diisopropyl-, Dibutyl- oder Diisobutylaminocarbonyl, Phenylaminocarbonyl,Methoxycarbonyl, Ethoxycarbonyl, Propoxycarbonyl, Isopropoxycarbonyl, Butoxycarbonyl, Isobutoxycarbonyl oder tert.Butoxycarbonyl steht,

B - für Sauerstoff oder Schwefel steht,

X - für eine Gruppe der Formel $-CH_2-CH_2-$ oder $-CH=CH-$steht, und

A - für eine Gruppe der Formel

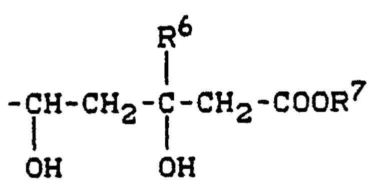 oder 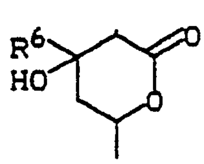 steht,

worin
$R^6$ - Wasserstoff, Methyl, Ethyl, Propyl, Isopropyl, Butyl, Isobutyl oder tert.Butyl bedeutet,
$R^7$ - Wasserstoff, Methyl, Ethyl, Propyl, Isopropyl, Butyl, Isobutyl, tert.Butyl oder Benzyl bedeutet, oder ein Natrium-, Kalium-, Calcium-, oder Magnesium- oder Ammoniumion bedeutet.

4. Substituierte Imidazolinone und Imidazolinthione gemäß Anspruch 1 zur therapeutischen Behandlung.

5. Verfahren zur Herstellung von substituierten Imidazolinonen und Imidazolinthionen der allgemeinen Formel

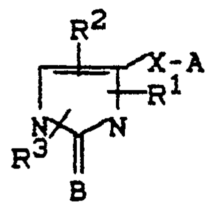

(I)

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl, oder durch eine Gruppe der Formel $-NR^4R^5$,
worin
$R^4$, $R^5$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,
worin
$R^4$ und $R^5$ die oben angegebene Bedeutung haben, oder
- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben,
$R^3$ - für Wasserstoff oder
- für Acyl, Alkylsulfonyl oder Arylsulfonyl steht, wobei der Arylrest durch Alkyl oder Halogen substituiert sein kann, oder
- für Aminocarbonyl, Alkylaminocarbonyl, Dialkylaminocarbonyl, Arylaminocarbonyl oder Alkoxycarbonyl steht, oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$ und $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
$R^3$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,
worin
$R^4$ und $R^5$ die oben angegebene Bedeutung haben,
oder
$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben
B - für Sauerstoff oder Schwefel steht,
X - für eine Gruppe der Formel $-CH_2-CH_2$ oder $-CH=CH-$steht, und
und
A - für eine Gruppe der Formel

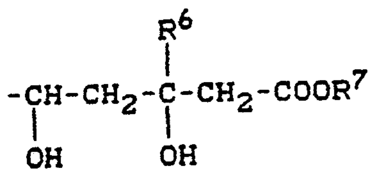

oder

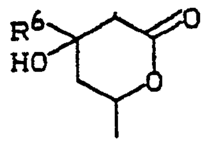

steht,

worin
$R^6$ - Wasserstoff oder Alkyl bedeutet
und
$R^7$ - Wasserstoff,
- einen Alkyl-, Aryl- oder Aralkylrest oder
- ein Kation bedeutet,
dadurch gekennzeichnet, daß man Ketone der allgemeinen Formel (VIII)

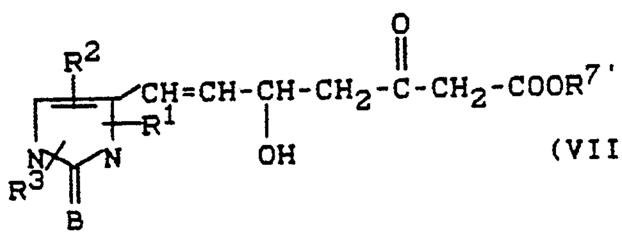

(VIII)

in welcher
$R^1$, $R^2$, $R^3$ und B die oben angegebene Bedeutung haben,
und
$R^{7'}$ - für Alkyl ($C_1$ bis $C_6$) Aryl ($C_6$ bis $C_{12}$) oder Aralkyl ($C_7$ bis $C_{10}$) steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen ($X = -CH_2-CH_2-$) die Ethenverbindungen ($X = -CH=CH-$) nach üblichen Methoden hydriert,
und gegebenenfalls Isomeren trennt.

6. Verfahren nach Anspruch 5, dadurch gekennzeichnet, daß die Reduktion im Temperaturbereich von -80 °C bis +30 °C durchgeführt wird.

7. Arzneimittel, enthaltend substituierte Imidazolinone und Imidazolinthione gemäß Anspruch 1.

8. Arzneimittel nach Anspruch 7, enthaltend 0,5 bis 90 Gew.-% der substituierten Imidazolinone und Imidazolinthione.

9. Verwendung von substituierten Imidazolinonen und Imidazolinthionen gemäß Anspruch 1 zur Behandlung von Krankheiten.

10. Verwendung von substituierten Imidazolinonen und Imidazolinthionen nach Anspruch 9 zur Behandlung von Hyperlipoproteinämie, Lipoproteinämie oder Arteriosklerose.

11. Verwendung von substituierten Imidazolinonen und Imidazolinthionen gemäß Anspruch 1 zur Herstellung von Arzneimitteln.

12. Ketone der allgemeinen Formel

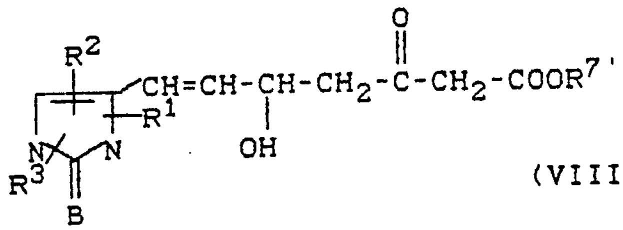

(VIII)

in welcher

R' - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$, $R^5$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifuormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin

$R^4$ und $R^5$ die oben angegebene Bedeutung haben,

oder

- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluor methoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

$R^3$ - für Wasserstoff oder

- für Acyl, Alkylsulfonyl oder Arylsulfonyl steht, wobei der Arylrest durch Alkyl oder Halogen substituiert sein kann, oder

- für Aminocarbonyl, Alkylaminocarbonyl, Dialkylaminocarbonyl, Arylaminocarbonyl oder Alkoxycarbonyl steht, oder

- für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$ und $R^5$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

$R^3$ - für Heteroaryl steht, das bis zu 3-fach gleich oder verschieden durch Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin
$R^4$ und $R^5$ die oben angegebene Bedeutung haben,
oder
$R^3$ - für Aryl steht, das bis zu 5-fach gleich oder verschieden durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio,Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben,
B - für Sauerstoff oder Schwefel steht,
X - für eine Gruppe der Formel $-CH_2-CH_2$ oder -CH = CH-steht,
und
$R^{7'}$ - für Alkyl, Aryl oder Aralkyl steht.

13. Verfahren zur Herstellung von Ketonen der allgemeinen Formel

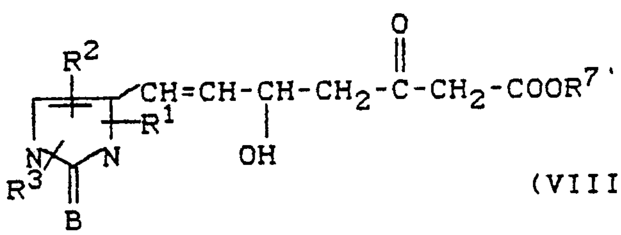

(VIII)

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$, $R^5$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifuormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,
worin
$R^4$ und $R^5$ die oben angegebene Bedeutung haben,
oder
$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben,
$R^3$ - für Wasserstoff oder
- für Acyl, Alkylsulfonyl oder Arylsulfonyl steht, wobei der Arylrest durch Alkyl oder Halogen substituiert sein kann, oder
- für Aminocarbonyl, Alkylaminocarbonyl, Dialkylaminocarbonyl, Arylaminocarbonyl oder Alkoxycarbonyl steht, oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin

$R^4$ und $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^3$ - für Heteroaryl steht, das bis zu 3-fach gleich oder verschieden durch Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,
worin
$R^4$ und $R^5$ die oben angegebene Bedeutung haben,
oder
$R^3$ - für Aryl steht, das bis zu 5-fach gleich oder verschieden durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio,Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben,
B - für Sauerstoff oder Schwefel steht,
X - für eine Gruppe der Formel $-CH_2-CH_2$ oder $-CH=CH$-steht,
und
$R^{7'}$ - für Alkyl, Aryl oder Aralkyl steht,
dadurch gekennzeichnet ist, daß man
Aldehyde der allgemeinen Formel (IX)

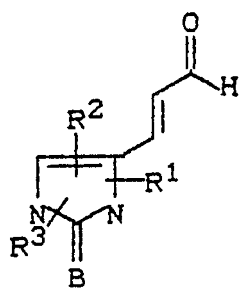

(IX)

in welcher
$R^1$, $R^2$, $R^3$ und B die oben angegebene Bedeutung haben,
in inerten Lösemitteln mit Acetessigester der allgemeinen Formel (X)

$$H_3C-\overset{O}{\overset{\|}{C}}-CH_2-COOR^{7'} \quad (X)$$

in welcher
$R^{7'}$ die oben angegebene Bedeutung hat,
in Anwesenheit von Basen umsetzt.

14. Aldehyde der allgemeinen Formel

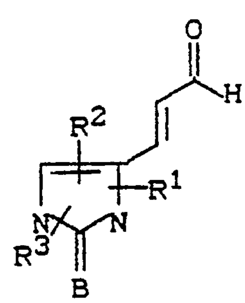

(IX)

in welcher
$R^1$ - für Cycloalkyl steht, oder
- für Alkyl steht, das substituiert sein kann durch Halogen, Cyano, Alkoxy, Alkylthio, Alkylsulfonyl,

Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$, $R^5$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Aryl thio, Arylsulfonyl, Trifluormethyl, Trifluormethoxy, Trifluormethylthio, Alkoxycarbonyl oder durch eine Gruppe der Formel $-NR^4R^5$ substituiert sein kann,
oder
$R^4$ und $R^5$ die oben angegebene Bedeutung haben,
oder
$R^2$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben
$R^3$ - für Wasserstoff oder
- für Acyl, Alkylsulfonyl oder Arylsulfonyl steht, wobei der Arylrest durch Alkyl oder Halogen substituiert sein kann, oder
- für Aminocarbonyl, Alkylaminocarbonyl, Dialkylaminocarbonyl, Arylaminocarbonyl oder Alkoxycarbonyl steht, oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$ und $R^5$ die oben angegebene Bedetutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder
$R^3$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder eine Gruppe der Formel $-NR^4R^5$ substituiert sein kann,
worin
$R^4$ und $R^5$ die oben angegebene Bedeutung haben,
oder
$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch durch eine Gruppe der Formel $-NR^4R^5$,
worin
$R^4$ und $R^5$ die oben angegebene Bedeutung haben, und
B für Sauerstoff oder Schwefel steht.

15. Verfahren zur Herstellung von Aldehyden der allgemeinen Formel

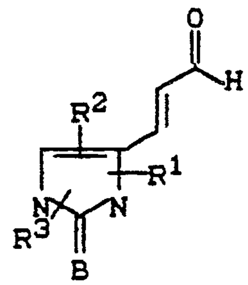

(IX)

in welcher
R' - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$, $R^5$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann, wobei
$R^4$ und $R^5$ die oben angegebene Bedeutung haben,
oder
- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben
$R^3$ - für Wasserstoff oder
- für Acyl, Alkylsulfonyl oder Arylsulfonyl steht, wobei der Arylrest durch Alkyl oder Halogen substituiert sein kann, oder
- für Aminocarbonyl, Alkylaminocarbonyl, Dialkylaminocarbonyl, Arylaminocarbonyl oder Alkoxycarbonyl steht, oder
- für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$ und $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder
$R^3$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder eine Gruppe der Formel $-NR^4R^5$ substituiert sein kann,
worin
$R^4$ und $R^5$ die oben angegebene Bedeutung haben
oder
$R^3$ - für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch durch eine Gruppe der Formel $-NR^4R^5$,

worin
$R^4$ und $R^5$ die oben angegebene Bedeutung haben, und
B für Sauerstoff oder Schwefel steht,
dadurch gekennzeichnet, daß man Imidazolinone und Imidazolinthione der allgemeinen Formel (XI)

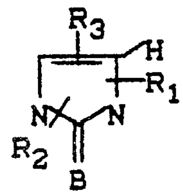

(XI)

in welcher
$R^1$, $R^2$, $R^3$ und B die oben angegebene Bedeutung haben,
in inerten Lösemitteln in Anwesenheit von Hilfsstoffen
mit N,N-Dialkylaminoacrolein der Formel (XII)

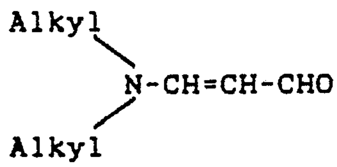

(XII),

wobei
Alkyl für einen geradkettigen oder verzweigten Kohlenwasserstoffrest mit 1 bis 6 Kohlenstoffatomen steht,
umsetzt.